# EUROPÄISCHE PATENTSCHRIFT

(11) **EP 1 500 427 B1**
(45) Veröffentlichungstag und Bekanntmachung des Hinweises auf die Patenterteilung: **09.04.2014**
(21) Anmeldenummer: 04016322.2
(22) Anmeldetag: 12.07.2004
(51) Int. Cl.: B01F 17/00, B01F 17/36, A61K 8/39, A61K 9/107, C08G 63/664

(54) **Emulgator für dünnflüssige W/O-Emulsionen auf Basis von teilvernetzten Polyglycerinestern der Polyhydroxystearinsäure**
Emulsifier for low-viscosity w/o emulsions, based on partially crosslinked polyglycerin esters of polyhydroxystearic acid.
Emulsifiant pour émulsions E/H de faible viscosité, à base d'esters partiellement réticulés de polyglycérine et d'acide polyhydroxystéarique.

(30) Priorität: 23.07.2003 DE 10333443
(43) Veröffentlichungstag der Anmeldung: 26.01.2005
(73) Patentinhaber: Evonik Degussa GmbH, 45128 Essen (DE)
(72) Erfinder: Allef, Petra, Dr., 45147 Essen (DE); Berkels, Wolfgang, 46239 Bottrop (DE); Dietz, Thomas, Dr., Glen Allen, Virginia 23060 (US); Grüning, Burghard, Dr., 45134 Essen (DE); Hameyer, Peter, 45138 Essen (DE)

(56) Entgegenhaltungen:
- EP-A1- 0 835 862
- EP-A2- 0 904 773
- DE-A1- 4 420 516
- DE-C1- 4 409 569

## Beschreibung

Die Erfindung betrifft Polyglycerinpartialester von Polyhydroxystearinsäure und mehrfunktionellen Carbonsäuren, erhältlich durch Veresterung eines Polyglyceringemisches mit Polyhydroxystearinsäure mit N = 1 bis 10 Einheiten und entweder Dimerfettsäuren, die durch katalytische Dimerisierung ungesättigter Fettsäuren mit 12 bis 22 C-Atomen erhalten wurden und eine mittlere Funktionalität von 2 bis 3 aufweisen, oder mit aliphatischen, linearen oder verzweigten Dicarbonsäuren mit einer Kettenlänge von 2 bis 16 C-Atomen und zusätzlich gesättigten oder ungesättigten, linearen oder verzweigten Fettsäuren mit 6 bis 22 C-Atomen, wobei der Veresterungsgrad der Polyglycerinmischung zwischen 20 und 75 % liegt. Die Erfindung umfasst deren Herstellung und Verwendung als W/O-Emulgatoren in kosmetischen oder pharmazeutischen Zubereitungen sowie als Hilfsmittel zur Dispergierung von anorganischen Mikropigmenten in Öldispersionen.

Es ist dem Fachmann bekannt, dass Wasser-in-Öl-Emulsionen aufgrund der dichten Packung der Wassertröpfchen, die eine Folge des üblicherweise hohen Anteils an disperser Phase (> 65 %) ist, schwer gegen Koaleszenz der Wassertröpfchen und damit gegen Wasserabscheidung zu stabilisieren sind. Eine weitere Folge der hohen Packungsdichte ist eine per se hohe Viskosität der Emulsion. Um ausreichende und von der kosmetischen Industrie geforderte Stabilität zu erzielen, verwendet man in der Regel stabilisierende Wachse und zum Teil auch hochviskose Öle, die beide die Viskosität der (äußeren) Ölphase erhöhen, somit die Mobilität der Wassertröpfchen reduzieren und gegen Koaleszenz schützen. Hochviskose Öle und stabilisierende Wachse haben jedoch einen negativen Einfluss auf das Hautgefühl, was sich in einem schweren und klebrigen Gefühl bemerkbar macht. Zwar kann man grundsätzlich auf Öl-in-Wasser-Emulsionen als Alternative ausweichen, die bekanntlich ein leichteres Hautgefühl besitzen, allerdings besitzen Wasser-in-Öl-Emulsionen einen deutlich besseren Pflegeeffekt als Öl-in-Wasser-Emulsionen.

Ein allgemein erstrebenswertes Ziel ist es also, Wasser-in-Öl-Emulsionen möglichst so leicht, d.h. niedrigviskos wie Öl-in-Wasser-Emulsionen zu machen, ohne deren besonderen Pflegeeffekt aufgeben zu müssen. Aufgabe ist es insbesondere, möglichst niedrigviskose, nicht klebrige und trotzdem stabile Wasser-in-Öl-Emulsionen zu ermöglichen. Hierbei kommt dem zu verwendenen Emulgator eine besondere Schlüsselrolle zu.

An W/O-Emulgatoren, die auf nativen Rohstoffen basieren, besteht aus ökologischen Gründen sowohl bei den Produzenten als auch bei den Konsumenten von Emulsionspräparaten ein erhebliches Interesse. Aus diesem Grunde finden Partialester aus Polyalkoholen, wie Glycerin, Polyglycerin, Sorbitol oder Methylglycosid und Fettsäuren, wie Öl- oder Isostearinsäure, eine vielfältige Verwendung.

Dieser Emulgatortyp ist jedoch zur Stabilisierung von fließfähigen Emulsionen (Lotionen) und Cremes mit hohem Gehalt an nativen Triglyceriden nicht geeignet. Die den Stabilitätsanforderungen des Marktes entsprechenden Cremes (Temperaturbelastbarkeit von -15 bis +45 °C, teilweise von -25 bis +50 °C) enthalten neben den genannten W/O-Emulgatoren an lipidartigen Komponenten überwiegend Paraffinöle und Fettsäureester von Monoalkanolen (MG < 500) ; diese haben günstigere technologische Eigenschaften als die höhermolekularen Triglyceride. Zur Stabilisierung werden dennoch zusätzlich relativ hohe Konzentrationen an viskositätserhöhenden Wachsen (≥ 3 %) benötigt, die sich negativ auf die Applikationseigenschaften auswirken, da sie ein unerwünschtes klebrig-fettiges Gefühl auf der Haut erzeugen.

Koemulgatoren, insbesondere Ethylenoxidaddukte in Kombination mit Metallseifen, erweitern den Anwendungsbereich lediglich auf paraffinölhaltige Lotionen.

Erheblich bessere Emulgiereigenschaften als die Polyalkohol-Fettsäurepartialester besitzen die Polyglycerinester dimerer und polymerisierter ungesättigter C₁₈-Fettsäuren. Sie werden aus den Mono- und Diglyceriden von Pflanzenölen, bevorzugt Sojaöl, durch eine mehrstündige thermische Behandlung bei ca. 300 °C bzw. durch die Umesterung eines thermisch polymerisierten Pflanzenöls mit Polyglycerin gewonnen.

Die nach einem analogen Verfahren aus Rizinusöl gebildeten Polyglycerin-Polyricinoleate sind ebenfalls leistungsfähige W/O-Emulgatoren (DE-B-44 09 569).

Beide Substanzklassen haben sich wegen ihrer Oxidationsempfindlichkeit und des oft fettig-ranzigen Geruchs nicht in kosmetischen oder pharmazeutischen Emulsionspräparaten etablieren können. Verantwortlich dafür sind in erster Linie die massive thermische Belastung während der Herstellung und der ungesättigte Charakter (Jodzahl ca. 100).

Das dem Polyglycerin-Polyricinoleat chemisch verwandte, ebenfalls aus vegetabilischen Rohstoffen herstellbare Polyglycerin-Polyhydroxystearat hat demgegenüber eine zufriedenstellende sensorische Qualität und besitzt die prinzipielle Befähigung zur Bildung cremeartiger und insbesondere fließfähiger W/O-Emulsionen.

Gemäß DE-A-40 12 693 wurden Ester von Polycarbonsäuren mit Polyhydroxyverbindungen vorgeschlagen. Durch Kondensation der Ausgangsverbindungen erhält man Produkte mit überschüssigen Carboxylgruppen, die anschließend neutralisiert werden. Die Reaktionsprodukte eignen sich als O/W-Emulgatoren.

In EP-B-0 835 862 werden Polyglycerinpartialester beschrieben, erhältlich durch Veresterung eines Polyglyceringemisches mit einem Veresterungsgrad des Polyglycerins zwischen 30 und 75 % und gesättigten oder ungesättigen, linearen oder verzweigten Fettsäuren mit 12 bis 22 C-Atomen und Dimerfettsäuren mit einer mittleren Funktionalität von 2 bis 2,4. Diese besitzen gegenüber dem Polyglycerin-Polyhydroxystearat den zusätzlichen Vorteil einer verbesserten Stabilität, insbesondere einer höheren Gefrier-Tau-Belastbarkeit der damit hergestellten W/O-Emulsionen. Die Emulsionen sind allerdings noch relativ dickflüssig und verursachen ein leicht klebriges Hautgefühl. Weitere Emulgatoren für W/O Emulsionen sind aus der EP 904 773 A2 bekannt.

Die Verbesserung der Gefrier-Tau-Belastbarkeit ist für die Transport- und Lagerfähigkeit der Emulsionspräparate von erheblichem praktischen Interesse. Durch längere Lagerung bei sehr tiefen Temperaturen oder durch extreme Temperaturschwankungen bei längeren Transportwegen kann sich die ungenügende Emulsionsstabilisierung durch deutliche Wasserabscheidungen im Emulsionspräparat zeigen oder sogar zur völligen Brechung der Emulsion führen.

Die Aufgabe der Erfindung bestand darin einen Emulgator zu entwickeln, der die Formulierung von dünnflüssigen Emulsionen erlaubt, die ein angenehmes Hautgefühl und gleichzeitig eine verbesserte Gefrier-Tau-Belastbarkeit aufweisen.

Es wurde nun überraschenderweise gefunden, dass insbesondere niedrigviskose, gleichzeitig aber besonders stabile Wasser-in-Öl-Emulsionen erhalten werden können, wenn man als Emulgatoren Polyglycerinpartialester von Polyhydroxystearinsäure, die zusätzlich mit Dicarbonsäuren verknüpft sind, verwendet.

Ein Gegenstand der Erfindung sind daher Polyglycerinpartialester von Polyhydroxystearinsäure und mehrfunktionellen Carbonsäuren, erhältlich durch Veresterung eines Polyglyceringemisches mit Polyhydroxystearinsäure mit N = 1 bis 10, vorzugsweise 2 bis 8, insbesondere 2 bis 5 Einheiten und Dimerfettsäuren, die durch katalytische Dimerisierung ungesättigter Fettsäuren mit 12 bis 22 C-Atomen erhalten wurden und eine mittlere Funktionalität von 2 bis 3 aufweisen und/oder mit aliphatischen, linearen oder verzweigten Di- und/oder Tricarbonsäuren mit einer Kettenlänge von 2 bis 16 C-Atomen und zusätzlich gesättigten oder ungesättigten, linearen oder verzweigten Fettsäuren mit 6 bis 22 C-Atomen, vorzugsweise 12 bis 18 C-Atomen, wobei der Veresterungsgrad der Polyglycerinmischung zwischen 20 und 75 %, vorzugsweise 40 bis 70 %, liegt.

Ein weiterer Gegenstand der Erfindung ist die Verwendung der erfindungsgemäßen Polyglycerinpartialester als W/O-Emulgatoren in kosmetischen oder pharmazeutischen Zubereitungen.

Überraschenderweise hat sich gezeigt, dass auch stark polare Pflanzenöle in stabile Emulsionen eingebracht werden können, während herkömmliche Emulgatoren bei diesen Produkten zu besonders bei niedrigen Temperaturen instabilen Emulsionen führen. Die Kondensationsprodukte auf Basis der 12-Hydroxystearinsäure sind flüssig und eignen sich somit neben der klassischen "Heiß-Heiß"-Herstellung auch zur energiesparenden "Kalt-Kalt"-Herstellung von Emulsionen.

Die Herstellung der erfindungsgemäß mitverwendeten Polyhydroxystearinsäuren erfolgt beispielsweise durch Polykondensation von Hydroxystearinsäure, vorzugsweise 12-Hydroxystearinsäure, die durch Härtung von Ricinolsäure bzw. technischer Ricinusölfettsäure gewonnen wird, nach den bekannten Verfahren. Es werden Veresterungsprodukte der allgemeinen Formel 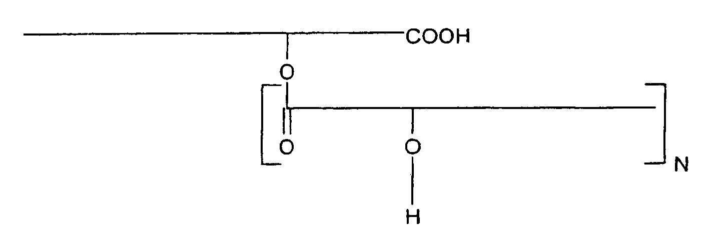 mit
- N =: 1 bis 10, vorzugsweise 2 bis 8, insbesondere 2 bis 5 Fettsäureeinheiten gebildet, die Säurezahlen zwischen 187 und 20 bevorzugt zwischen 96 und 45 aufweisen.

Als Polyglycerine sind insbesondere solche der allgemeinen Formel 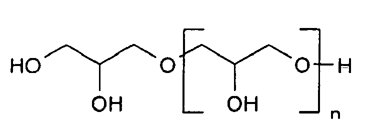 mit einem mittleren Kondensationsgrad n von 1 bis 11, bevorzugt 2 bis 6, insbesondere bevorzugt 3 bis 6 und Hydroxylzahlen von ca. 1.350 bis ca. 800, bevorzugt ca. 1.200 bis ca. 900 geeignet.

Dabei handelt es sich um technische Polyglycerinmischungen, die z. B. durch alkalisch katalysierte Kondensation von Glycerin bei erhöhten Temperaturen erhalten werden, aus denen sich gegebenenfalls durch Destillationsverfahren Fraktionen mit dem erwünschten Kondensationsgrad erhalten lassen. Ebenfalls geeignet sind auch PoIyglycerine, die auf anderem Wege, z. B. aus Epichlorhydrin oder Glycidol gewonnen werden.

Als besonders vorteilhaft hat sich die Verwendung von Polyglycerinen erwiesen, die die folgende Homologenverteilung (GC-Methode) aufweisen; in Klammern angegeben sind die bevorzugten Bereiche:
Glycerin: 0 bis 20 (< 5) Gew.-%
Diglycerine: 0 bis 60 (5 bis 30) Gew.-%
Triglycerine: 0 bis 60 (5 bis 50) Gew.-%
Tetraglycerine: 0 bis 30 (5 bis 25) Gew.-%
Pentaglycerine: 0 bis 30 (5 bis 20) Gew.-%
Oligoglycerine: ad 100 Gew.-%

Bei den eingesetzten Dimerfettsäuren handelt es sich bekanntlich um ein Gemisch aus acyclischen und cyclischen Dicarbonsäuren, die durch eine katalysierte Dimerisierung ungesättigter Fettsäuren mit 12 bis 22 C-Atomen erhalten werden und eine mittlere Funktionalität von 2 bis 3, vorzugsweise ca. 2 aufweisen. Sie können auch in untergeordnetem Ausmaß polymere Fettsäuren (trimer- und höher funktionelle) enthalten. Die Säurezahlen liegen im Bereich von 150 bis 290 bevorzugt 190 bis 200.

Handelsübliche Produkte haben im Durchschnitt

| | |
|---|---|
| Monomergehalte von ca. | 7 bis 15 Gew.-% |
| Dimergehalte von ca. | 70 bis 77 Gew.-% |
| Polymergehalte von ca. | 15 bis 16 Gew.-%. |

Sie können durch die bekannten Trennungsverfahren auf höhere Gehalte der jeweiligen Funktionalitäten (Mono, Di, Tri) und/oder durch Hydrierung auf niedrige Anteile ungesättigter Fettsäuren (niedrige Jodzahlen) eingestellt werden.

Weitere Informationen sind den Produktblättern der Hersteller wie beispielsweise der Firmen Uniqema ( Pripol® ) und Arizona Chem. (Unidyme® , Century® ) zu entnehmen.

Zur Herstellung und Verwendung von Dimersäuren und deren physikalischen und chemischen Eigenschaften wird auch auf die Veröffentlichung "The Dimer Acids: The chemical and physical properties, reactions and applications", Ed. E.C. Leonard; Humko Sheffield Chemical, 1975, Memphis, Tenn., verwiesen.

Besonders geeignet für den erfindungsgemäßen Einsatzzweck als Emulgatoren ist die Verwendung von relativ kurzkettigen Di- oder Tricarbonsäuren anstelle von Dimersäuren, wie Oxalsäure, Malonsäure, Bernsteinsäure, Glutarsäure, Adipinsäure, Pimelinsäure, Korksäure, Azelainsäure, Sebacinsäure, Dodecandisäure. Ebenso geeignet sind Hydroxydi- bzw. -tricarbonsäuren, wie Äpfelsäure, Weinsäure, Zitronensäure.

Insbesondere bevorzugt sind die Alkandicarbonsäuren mit 4 bis 14 C-Atomen.

Durch Mitverwendung dieser Säuren kann nochmals eine deutlich verbesserte Stabilisierung der Phasengrenzflächen in W/O-Emulsionen erzielt werden.

Als zusätzliche Fettsäurekomponenten eignen sich vor allem gesättigte Fettsäuren, wie Laurinsäure, Tridecansäure, Myristinsäure, Palmitinsäure, Margarinsäure, Stearinsäure, Isostearinsäure, Arachinsäure und Behensäure sowie Mischungen hieraus.

Natürlich vorkommende Mischungen sind beispielsweise die Kokosfettsäuren, die als Hauptbestandteil Laurinsäure, daneben noch gesättigte C₁₄- bis C₁₈-Fettsäuren und gegebenenfalls gesättigte C₈- bis C₁₀-Fettsäuren und ungesättigte Fettsäuren enthalten, sowie Talgfettsäuren, welche im wesentlichen eine Mischung aus Palmitinsäure und Stearinsäure darstellen.

Als zusätzliche ungesättigte Fettsäurekomponenten eignen sich monoolefinisch ungesättigte Säuren, beispielsweise Hexadecensäuren, Octadecensäuren, wie Ölsäure (cis-9-Octadecensäure) oder Elaidinsäure (trans-9-Octadecensäure), Eicosensäuren und Docosensäuren, wie Erucasäure (cis-13-Docosensäure) oder Brassidinsäure (trans-13-Docosensäure), mehrfach ungesättigte Fettsäuren, beispielsweise Octadecadiensäuren und Octadecatriensäuren, wie Linolsäure und Linolensäure, sowie Mischungen hieraus.

Besonders geeignet sind die flüssigen Fettsäuren wie Öl-, Ricinol-, Eruca- und Isostearinsäure, die 18 bis 22 Kohlenstoffatome enthalten. Ihre Erstarrungspunkte liegen aufgrund einer Verzweigung oder einer Doppelbindung in der Kohlenwasserstoffkette unter 35 °C. Verwendbar sind weiterhin Fettsäuregemische, die auch wachsartige Komponenten, wie gehärtete Ricinolsäure, enthalten können.
Desweiteren ist die Verwendung von Lactonen, wie Butyrolacton oder Caprolacton als Fettsäurekomponente möglich.

In den erfindungsgemäßen Polyglycerinpartialestern sind die Hydroxylgruppen des Polyglycerins zu 20 bis 75 %, bevorzugt 40 bis 70 %, verestert.

Bei ihrer Herstellung werden die Varianten A) und B) bevorzugt gemäß denen in einer
A) ersten Stufe Polyglycerin bis zu einem Veresterungsgrad von 10 bis 70 %, bevorzugt 25 bis 40 %, mit Fettsäure und Dicarbonsäure und/oder Dimersäure verestert und in einer zweiten Stufe mit Polyhydroxystearinsäure zu einem Gesamtveresterungsgrad 20 bis 75 %, bevorzugt 40 bis 60 %, verestert oder in einer
B) ersten Stufe Polyglycerin bis zu einem Veresterungsgrad von 10 bis 70 %, bevorzugt 25 bis 40 %, mit Fettsäure und Polyhydroxystearinsäure verestert und in einer
   zweiten Stufe mit Dicarbonsäure und/oder Dimersäure zu einem Gesamtveresterungsgrad 20 bis 75 %, bevorzugt 40 bis 60 %, verestert.

Andere Zugabereihenfolgen der Fettsäuren und Eintopfverfahren sind ebenfalls möglich.

Das erfindungsgemäße molare Verhältnis Polyhydroxystearinsäure zu Disäure/Dimersäure zu Fettsäure liegt im Bereich 0,25 bis 4 mol zu 0,1 bis 2 mol zu 0 bis 10 mol, vorzugsweise im Bereich 0,25 bis 2 mol zu 0,3 bis 1 mol zu 0,5 bis 3 mol, wobei durch geeignete Auswahl der hydrophilen und lipophilen Molekülanteile ein HLB-Wert von ca. 3 bis 8 eingestellt wird, um für die Stabilisierung von W/O-Emulsionen günstige Eigenschaften zu erhalten. Dies wird erreicht, indem der Anteil hydrophiler Komponenten im Bereich 10 bis 70 % liegt, bevorzugt 20 bis 40 %.

Die erfindungsgemäßen Polyglycerinpartialester können in an sich bekannter Weise durch Erhitzen der Reaktionskomponenten und destillativer Abtrennung des entstehenden Reaktionswassers hergestellt werden. Zur Beschleunigung können saure oder basische Katalysatoren, wie Sulfonsäuren, Phosphorsäure oder phosphorige Säure, Lewissäuren, wie Zinnsalze, Alkali- oder Erdalkalimetalloxide oder -hydroxide, -alkoholate oder -salze mitverwendet werden. Der Zusatz eines Katalysators ist aber nicht unbedingt notwendig. Der fortschreitende Umsatz kann beispielsweise über das abgetrennte Reaktionswasser, durch Messung der Säurezahl oder durch Infrarotspektroskopie verfolgt werden. Im allgemeinen wird eine Säurezahl im Endprodukt von < 20, bevorzugt < 10 angestrebt. Besonders bevorzugt sind Produkte mit einer Säurezahl < 5.

Die erfindungsgemäßen Polyglycerinester eignen sich zur Stabilisierung von wässrigen Emulsionen und Dispersionen.

Bevorzugt ist ihre Verwendung als Emulgatoren zur Herstellung kosmetischer oder pharmazeutischer Zubereitungen. In Frage kommende kosmetische Zubereitungen, die durch Verwendung von Öl-in-Wasser- oder Wasser-in-Öl-Emulgatoren eine leicht streichbare Konsistenz erhalten, weil sich durch diese Emulgatorsysteme ein Öl oder ein Fett gut in eine wässrige Phase bzw. eine wässrige Phase gut in ein Öl oder ein Fett einarbeiten lässt, beispielsweise Cremes, wie Pflegecremes, Babycremes oder Sonnenschutzcremes, Salben, Lotionen oder Schminken. In pharmazeutischen Zubereitungen, wie Salben oder Cremes, benötigt man Öl-in-Wasser- oder Wasser-in-Öl-Emulgatoren zur Applikation von Wirkstoffen.

Als Ölkomponente können eingesetzt werden:
- Esteröle, wie Octylpalmitat, Octylstearat, Cetyloctanoat, Capryl/Caprinsäuretriglyceride, Decyloleat, Cetearyloctanoat, Isopropylmyristat, Isopropylpalmitat, C₁₂₋₁₃-Alkylbenzoat, Stearylheptanoat, Dibutyladipate
- Pflanzenöle, wie Weizenkeimöl, Jojobaöl, Olivenöl, Borretschöl, Avocadoöl, Erdnussöl, Mandelöl, Sonnenblumenöl, Walnussöl
- höhere Alkohole, wie Octyldodecanol (oder Guerbet-Alkohole allgemein), Oleylalkohol, und
- paraffinartige (Kohlenwasserstoff)Öle, wie Paraffinöl, Isohexadecane, Polydecene, Vaseline, Paraffinum perliquidum, Squalan.

Weiterhin werden die dem Fachmann bekannten in den kosmetischen und pharmazeutischen Anwendungen üblichen Zusammensetzungen und Bestandteile sowie übliche Hilfsmittel und Zusatzstoffe, wie Stabilisatoren oder Konservierungsmittel, für derartige kosmetische und pharmazeutische Zubereitungen mitverwendet.

Die erfindungsgemäß verwendeten Polyglycerinpartialester bewirken gegenüber dem Stand der Technik bei tiefen Temperaturen eine deutlich erhöhte Lagerstabilität der mit ihnen hergestellten kosmetischen und pharmazeutischen Zubereitungen, was bei im Vergleich zu Öl-in-Wasser-Emulsionen schwerer zu stabilisierenden Wasser-in-Öl-Emulsionen besonders bedeutsam ist.

Weiterhin benötigt man in der Regel von den Polyglycerinpartialestern geringere Konzentrationen und/oder geringere Mengen von konsistenzgebenden Wachsen in den Zubereitungen, um eine gleiche Wirkung wie mit Mitteln des Standes der Technik zu erzielen. Die mit den erfindungsgemäßen Polyglycerinpartialestern hergestellten Wasser-in-Öl-Emulsionen sind zudem dünnflüssiger als ähnlich stabile Emulsionen, die mit Emulgatoren vom Stand der Technik hergestellt sind.

### Synthesebeispiele:

### Beispiel 1:

In erster Stufe wird Polyglycerin (Hydroxylzahl (OH-Z) = 1.090) mit Fettsäure verestert.
88,4 g (0,31 mol) Isostearinsäure und 157,5 g (0,13 mol) Polyhydroxystearinsäure mit einer Säurezahl (SZ) von 47 werden mit 100 g Polyglycerin in der ersten Stufe bei 240 °C und unter Stickstoffdurchleitung verestert. Nach 2 Stunden Reaktionszeit bei dieser Temperatur betrug die Säurezahl < 10. In zweiter Stufe wird auf 130 °C heruntergekühlt und 22,5 g (0,11 mol) Sebacinsäure zugegeben. Der Ansatz wird wieder auf 240 °C erhitzt und bei dieser Temperatur 3 Stunden gerührt. Es wird ein viskoses Produkt mit einer Säurezahl von < 5 erhalten.

### Beispiel 2:

91,1 g (0,32 mol) Isostearinsäure und 141,7 g (0,12 mol) Polyhydroxystearinsäure mit einer Säurezahl (SZ) von 47 werden mit 61,9 g Polyglycerin (OH-Z = 950) bei 240 °C und unter Stickstoffdurchleitung verestert. Nach 2 Stunden Reaktionszeit bei dieser Temperatur betrug die Säurezahl < 10. In zweiter Stufe wird auf 130 °C heruntergekühlt und 20,2 g (0,1 mol) Sebacinsäure zugegeben. Der Ansatz wird wieder auf 240 °C erhitzt und bei dieser Temperatur 3 Stunden gerührt. Es wird ein viskoses Produkt mit einer Säurezahl von < 5 erhalten.

### Beispiel 3:

120,0 g (0,42 mol) Isostearinsäure und 75,0 g (0,17 mol) Polyhydroxystearinsäure mit einer Säurezahl (SZ) von 47 werden mit 75,0 g Polyglycerin (OH-Z = 950) bei 240 °C und unter Stickstoffdurchleitung verestert. Nach 2 Stunden Reaktionszeit bei dieser Temperatur betrug die Säurezahl < 10. In zweiter Stufe wird auf 130 °C heruntergekühlt und 20,2 g (0,1 mol) Sebacinsäure zugegeben. Der Ansatz wird wieder auf 240 °C erhitzt und bei dieser Temperatur 3 Stunden gerührt. Es wird ein viskoses Produkt mit einer Säurezahl von < 5 erhalten.

### Beispiel 4:

111,8 g (0,39 mol) Isostearinsäure und 110,4 g (0,09 mol) Polyhydroxystearinsäure mit einer Säurezahl (SZ) von 47 werden mit 61,9 g Polyglycerin (OH-Z = 1.090) bei 240 °C und unter Stickstoffdurchleitung verestert. Nach 2 Stunden Reaktionszeit bei dieser Temperatur betrug die Säurezahl < 10. In zweiter Stufe wird auf 130 °C heruntergekühlt und 20,2 g (0,1 mol) Sebacinsäure zugegeben. Der Ansatz wird wieder auf 240 °C erhitzt und bei dieser Temperatur 3 Stunden gerührt. Es wird ein viskoses Produkt mit einer Säurezahl von < 5 erhalten.

### Beispiel 5:

91,1 g (0,32 mol) Isostearinsäure und 141,7 g (0,12 mol) Polyhydroxystearinsäure mit einer Säurezahl (SZ) von 45 werden mit 61,9 g Polyglycerin (OH-Z = 1.090) in der ersten Stufe bei 240 °C und unter Stickstoffdurchleitung verestert. Nach 2 Stunden Reaktionszeit bei dieser Temperatur betrug die Säurezahl < 10. Anschließend wird auf 180 °C heruntergekühlt und 57,5 g (0,1 mol) Dimerfettsäure (Empol 1.062) zugegeben. Der Ansatz wird wieder auf 240 °C erhitzt und bei dieser Temperatur 3 Stunden gerührt. Es wird ein viskoses Produkt erhalten, das durch Säurezahl von < 5 gekennzeichnet ist.

### Beispiel 6:

91,1 g (0,32 mol) Isostearinsäure und 141,7 g (0,12 mol) Polyhydroxystearinsäure mit einer Säurezahl (SZ) von 45 werden mit 61,9 g Polyglycerin (OH-Z = 1.090) in der ersten Stufe bei 240 °C und unter Stickstoffdurchleitung verestert. Nach 2 Stunden Reaktionszeit bei dieser Temperatur betrug die Säurezahl < 10. Anschließend wird auf 130 °C heruntergekühlt und 14,6 g (0,1 mol) Adipinsäure zugegeben. Der Ansatz wird wieder auf 240 °C erhitzt und bei dieser Temperatur 3 Stunden gerührt. Es wird ein viskoses Produkt erhalten, das durch eine Säurezahl von < 5 gekennzeichnet ist.

### Beispiel 7:

88,4 g (0,31 mol) Isostearinsäure und 22,5 g (0,11 mol) Sebacinsäure werden mit 175,8 g Polyglycerin (OH-Z = 850) in der ersten Stufe bei 240 °C unter Stickstoffdurchleitung verestert. Nach 2 Stunden Reaktionszeit bei dieser Temperatur betrug die Säurezahl < 10. Dann werden bei 240 °C unter Stickstoffdurchleitung 157,5 g (0,13 mol) Polyhydroxystearinsäure mit einer Säurezahl (SZ) von 45 zugegeben. Anschließend wird der Ansatz solange bei 240 °C gerührt, bis die SZ < 5 beträgt.

### Beispiel 8:

91,1 g (0,32 mol) Isostearinsäure und 20,2 g (0,1 mol) Sebacinsäure werden mit 61,9 g Polyglycerin (OH-Z = 1090) in der ersten Stufe bei 240 °C unter Stickstoffdurchleitung verestert. Nach 2 Stunden Reaktionszeit bei dieser Temperatur betrug die Säurezahl < 10. Dann werden bei 240 °C unter Stickstoffdurchleitung 141,7 g (0,12 mol) Polyhydroxystearinsäure mit einer Säurezahl (SZ) von 59 zugegeben. Anschließend wird der Ansatz solange bei 240 °C gerührt, bis die SZ < 5 beträgt.

### Beispiel 9:

215,6 g (0,2 mol) Polyhydroxystearinsäure mit einer Säurezahl (SZ) von 48, welche zuvor mit einer äquivalenten Menge an Isostearinsäure verestert wurde, werden mit 24 g Polyglycerin (OH-Z = 1.190) in an sich bekannter Weise verestert. Nach 2 Stunden Reaktionszeit bei dieser Temperatur betrug die Säurezahl < 10. Anschließend wird auf 130 °C heruntergekühlt und 10,1 g (0,2 mol) Sebacinsäre zugegeben. Der Ansatz wird wieder auf 240 °C erhitzt und bei dieser Temperatur 8 Stunden gerührt, bis eine Säurezahl von < 5 erreicht ist.

### Beispiel 10:

Die Herstellung des Polyglycerinesters erfolgt in 1 Stufe.
61 g Polyglycerin (OH-Z = 1.090), 91,1 g (0,32 mol) Isostearinsäure, 20,2 g (0,1 mol) Sebacinsäure sowie 141,7 g (0,12 mol) Polyhydroxystearinsäure mit einer Säurezahl (SZ) von 59 werden zusammen vorgelegt und dann unter Stickstoffdurchleitung bis auf 240 °C solange erhitzt, bis die Säurezahl < 5 beträgt.

### Beispiel 11:

91,1 g (0,32 mol) Isostearinsäure, 10,1 g (0,05 mol) Sebacinsäure und 6,7 g (0,05 mol) Äpfelsäure werden mit 61,9 g Polyglycerin (OH-Z = 1.090) in der ersten Stufe bei 240 °C unter Stickstoffdurchleitung verestert. Nach 2 Stunden Reaktionszeit bei dieser Temperatur betrug die Säurezahl < 10. Dann werden bei 240 °C unter Stickstoffdurchleitung 141,7 g (0,12 mol) Polyhydroxystearinsäure mit einer Säurezahl (SZ) von 45 zugegeben. Anschließend wird der Ansatz solange bei 240 °C gerührt, bis die SZ < 5 beträgt.

### Beispiel 12:

91,1 g (0,32 mol) Isostearinsäure und 20,2 g (0,1 mol) Sebacinsäure werden mit 61,9 g Polyglycerin (OH-Z = 850) in der ersten Stufe bei 240 °C unter Stickstoffdurchleitung verestert. Nach 2 Stunden Reaktionszeit bei dieser Temperatur betrug die Säurezahl < 10. Dann werden bei 240 °C unter Stickstoffdurchleitung 141,7 g (0,12 mol) Polyhydroxystearinsäure mit einer Säurezahl (SZ) von 45 zugegeben. Anschließend wird der Ansatz solange bei 240 °C gerührt, bis die SZ < 5 beträgt.

### Beispiel 13:

91,1 g (0,32 mol) Isostearinsäure und 20,2 g (0,1 mol) Sebacinsäure werden mit 63 g Polyglycerin (OH-Z = 1190) in der ersten Stufe bei 240 °C unter Stickstoffdurchleitung verestert. Nach 2 Stunden Reaktionszeit bei dieser Temperatur betrug die Säurezahl < 10. Dann werden bei 240 °C unter Stickstoffdurchleitung 141,7 g (0,12 mol) Polyhydroxystearinsäure mit einer Säurezahl (SZ) von 45 zugegeben. Anschließend wird der Ansatz solange bei 240 °C gerührt, bis die SZ < 5 beträgt.

### Beispiel 14:

88,4 g (0,31 mol) Isostearinsäure und 22,5 g (0,11 mol) Sebacinsäure werden mit 127,8 g Polyglycerin (OH-Z = 890) in der ersten Stufe bei 240 °C unter Stickstoffdurchleitung verestert. Nach 2 Stunden Reaktionszeit bei dieser Temperatur betrug die Säurezahl < 10. Dann werden bei 240 °C unter Stickstoffdurchleitung 157,5 g (0,12 mol) Polyhydroxystearinsäure mit einer Säurezahl (SZ) von 45 zugegeben. Anschließend wird der Ansatz solange bei 240 °C gerührt, bis die SZ < 5 beträgt.

### Beispiel 15:

88,4 g (0,31 mol) Isostearinsäure und 22,5 g (0,11 mol) Sebacinsäure werden mit 175,8 g Polyglycerin (OH-Z = 850) in der ersten Stufe bei 240 °C unter Stickstoffdurchleitung verestert. Nach 2 Stunden Reaktionszeit bei dieser Temperatur betrug die Säurezahl < 10. Dann werden bei 240 °C unter Stickstoffdurchleitung 157,5 g (0,13 mol) Polyhydroxystearinsäure mit einer Säurezahl (SZ) von 45 zugegeben. Anschließend wird der Ansatz solange bei 240 °C gerührt, bis die SZ < 5 beträgt.

### Beispiel 16:

132,9 g (0,47 mol) Isostearinsäure und 16,9 g (0,08 mol) Sebacinsäure werden mit 51,6 g Polyglycerin (OH-Z = 1.080) in der ersten Stufe bei 240 °C unter Stickstoffdurchleitung verestert. Nach 2 Stunden Reaktionszeit bei dieser Temperatur betrug die Säurezahl < 10. Dann werden bei 240 °C unter Stickstoffdurchleitung 237,7 g (0,2 mol) Polyhydroxystearinsäure mit einer Säurezahl (SZ) von 45 zugegeben. Anschließend wird der Ansatz solange bei 240 °C gerührt, bis die SZ < 5 beträgt.

### Formulierungsbeispiele:

### Formulierungsbeispiel 1:

Als Emulgator wird ein Emulgator vom Stand der Technik auf Basis von Polyglycerinpartialestern aus Fettsäure und Dimersäure (EP-B-0 835 862) verwendet.

| | **Formulierung 1A (%)** | **Formulierung 1B (%)** |
|---|---|---|
| **A** Emulgator nach Stand der Technik | 2,50 | 2,50 |
| Castorwachs | 0,25 | 0,25 |
| Ceresin W 80 | 0,25 | 0,25 |
| Paraffinöl | - | 14,00 |
| Octylpalmitat | 11,00 | 8,00 |
| C_{8/10}-Triglycerin | 11,00 | - |
| **B** Glycerin | 3,35 | 3,35 |
| Bronopol | 0,05 | 0,05 |
| MgSO₄ x 7 H₂O | 0,60 | 0,60 |
| Wasser | 71,00 | 71,00 |

### Herstellung:

B (20 °C) in A (80 °C) einrühren, homogenisieren, nach 2 h Abkühlzeit Homogenisierung wiederholen.

### Formulierungsbeispiel 2:

Als Emulgator wird ein anderer Emulgator vom Stand der Technik auf Basis von Polyglycerinpartialestern der Polyhydroxystearinsäure (DE-A-44 09 569) verwendet.

| | **Formulierung 2A (%)** | **Formulierung 2B (%)** |
|---|---|---|
| **A** Emulgator nach Stand der Technik | 2,50 | 2,50 |
| Castorwachs | 0,25 | 0,25 |
| Ceresin W 80 | 0,25 | 0,25 |
| Paraffinöl | - | 14,00 |
| Octylpalmitat | 11,00 | 8,00 |
| C_{8/10}-Triglycerin | 11,00 | - |
| **B** Glycerin | 3,35 | 3,35 |
| Bronopol | 0,05 | 0,05 |
| MgSO₄ x 7 H₂O | 0,60 | 0,60 |
| Wasser | 71,00 | 71,00 |

### Herstellung:

B (20 °C) in A (80 °C) einrühren, homogenisieren, nach 2 h Abkühlzeit Homogenisierung wiederholen.

### Formulierungsbeispiel 3:

Als Emulgator wird ein Emulgator nach Ausführungsbeispiel 1 verwendet.

| | **Formulierung 3A (%)** | **Formulierung 3B (%)** |
|---|---|---|
| **A** Emulgator nach Ausführungsbsp. 1 | 2,50 | 2,50 |
| Castorwachs | 0,25 | 0,25 |
| Ceresin W 80 | 0,25 | 0,25 |
| Paraffinöl | - | 14,00 |
| Octylpalmitat | 11,00 | 8,00 |
| C_{8/10}- Triglycerin | 11,00 | - |
| **B** Glycerin | 3,35 | 3,35 |
| Bronopol | 0,05 | 0,05 |
| MgSO₄ x 7 H₂O | 0,60 | 0,60 |
| Wasser | 71,00 | 71,00 |

### Herstellung:

B (20 °C) in A (80 °C) einrühren, homogenisieren, nach 2 h Abkühlzeit Homogenisierung wiederholen.

### Formulierungsbeispiel 4:

Als Emulgator wird ein Emulgator nach Ausführungsbeispiel 2 verwendet.

| | **Formulierung 4A (%)** | **Formulierung 4B (%)** |
|---|---|---|
| **A** Emulgator nach Ausführungsbsp. 2 | 2,50 | 2,50 |
| Castorwachs | 0,25 | 0,25 |
| Ceresin W 80 | 0,25 | 0,25 |
| Paraffinöl | - | 14,00 |
| Octylpalmitat | 11,00 | 8,00 |
| C_{8/10}-Triglycerin | 11,00 | - |
| **B** Glycerin | 3,35 | 3,35 |
| Bronopol | 0,05 | 0,05 |
| MgSO₄ x 7 H₂O | 0,60 | 0,60 |
| Wasser | 71,00 | 71,00 |

### Herstellung:

B (20 °C) in A (80 °C) einrühren, homogenisieren, nach 2 h Abkühlzeit Homogenisierung wiederholen.

### Formulierungsbeispiel 5:

Als Emulgator wird ein Emulgator nach Ausführungsbeispiel 3 verwendet.

| | **Formulierung 5A %** | **Formulierung 5B %** |
|---|---|---|
| **A** Emulgator nach Ausführungsbsp. 3 | 2,50 | 2,50 |
| Castorwachs | 0,25 | 0,25 |
| Ceresin W 80 | 0,25 | 0,25 |
| Paraffinöl | - | 14,00 |
| Octylpalmitat | 11,00 | 8,00 |
| C_{8/10}-Triglycerin | 11,00 | - |
| **B** Glycerin | 3,35 | 3,35 |
| Bronopol | 0,05 | 0,05 |
| MgSO₄ x 7 H₂O | 0,60 | 0,60 |
| Wasser | 71,00 | 71,00 |

### Herstellung:

B (20 °C) in A (80 °C) einrühren, homogenisieren, nach 2 h Abkühlzeit Homogenisierung wiederholen.

### Formulierungsbeispiel 6:

Als Emulgator wird ein Emulgator nach Ausführungsbeispiel 4 verwendet.

| | **Formulierung 6A (%)** | **Formulierung 6B (%)** |
|---|---|---|
| **A** Emulgator nach Ausführungsbsp. 4 | 2,50 | 2,50 |
| Castorwachs | 0,25 | 0,25 |
| Ceresin W 80 | 0,25 | 0,25 |
| Paraffinöl | - | 14,00 |
| Octylpalmitat | 11,00 | 8,00 |
| C_{8/10}-Triglycerin | 11,00 | - |
| **B** Glycerin | 3,35 | 3,35 |
| Bronopol | 0,05 | 0,05 |
| MgSO₄ x 7 H₂O | 0,60 | 0,60 |
| Wasser | 71,00 | 71,00 |

### Herstellung:

B (20 °C) in A (80 °C) einrühren, homogenisieren, nach 2 h Abkühlzeit Homogenisierung wiederholen.

Während die Emulsionen 3A, 3B, 4A, 4B, 5A, 5B, 6A und 6B hergestellt mit einem erfindungsmäßigen Emulgator, eine niedrige Viskosität (Brookefield RVT Helipath Stand, Modell D; V.A.C. 230, Spindel C) besitzen, zeigen Emulsion 1A und 1B, hergestellt mit einem Emulgator vom Stand der Technik auf Basis von Polyglycerinpartialestern aus Fettsäure und Dimersäure (EP-B-0 835 862), eine erhöhte Viskosität und damit ein leicht klebriges Hautgefühl.

Die Emulsionen 2A und 2B, hergestellt mit einem Emulgator vom Stand der Technik auf Basis von Polyglycerinpartialestern der Polyhydroxystearinsäure (DE-A-44 09 569), zeigen zwar eine mittlere Viskosität aber eine deutliche Wasserabscheidung nach Tieftemperaturlagerung (12 h bei -15 °C bzw. -25 °C), was einen Transport der Emulsionen erschwert.

Die Ergebnisse sind in der folgenden Tabelle zusammengefasst:

| | **Viskosität [Pas]** | **Stabilität** |
|---|---|---|
| **Emulsion 1A** | 28 | stabil |
| **Emulsion 1B** | 23 | stabil |
| **Emulsion 2A** | 19 | Wasserabscheidung bei -15 °C |
| **Emulsion 2B** | 15 | Wasserabscheidung bei -25 °C |
| **Emulsion 3A** | 13 | stabil |
| **Emulsion 3B** | 8 | stabil |
| **Emulsion 4A** | 13 | stabil |
| **Emulsion 4B** | 10 | stabil |
| **Emulsion 5A** | 12 | stabil |
| **Emulsion 5B** | 10 | stabil |
| **Emulsion 6A** | 11 | stabil |
| **Emulsion 6B** | 9 | stabil |

## Patentansprüche

1. Polyglycerinpartialester von Polyhydroxystearinsäure und mehrfunktionellen Carbonsäuren, erhältlich durch Veresterung eines
a) Polyglyceringemisches mit
b) Polyhydroxystearinsäure und
c) Di- und/oder Tricarbonsäuren und gegebenenfalls/oder mit
d) Dimerfettsäuren, und
e) Fettsäuren mit 6 bis 22 C-Atomen.

2. Polyglycerinpartialester gemäß Anspruch 1, **dadurch gekennzeichnet, dass** das Polyglyceringemisch gemäß a) einen mittleren Kondensationsgrad von 1 bis 10 aufweist.

3. Polyglycerinpartialester gemäß den Ansprüchen 1 bis 2, **dadurch gekennzeichnet, dass** die Polyhydroxystearinsäure gemäß b) einen mittleren Kondensationsgrad von 1 bis 10 aufweist.

4. Polyglycerinpartialester gemäß den Ansprüchen 1 bis 3, **dadurch gekennzeichnet, dass** die Di- und/oder Tricarbonsäuren gemäß c) aliphatische, lineare oder verzweigte Dicarbonsäuren sind und 2 bis 16 C-Atome aufweisen.

5. Polyglycerinpartialester gemäß den Ansprüchen 1 bis 4, **dadurch gekennzeichnet, dass** die Dimerfettsäuren gemäß d) eine mittlere Funktionalität von 2 bis 3 aufweisen.

6. Polyglycerinpartialester gemäß den Ansprüchen 1 bis 5, **dadurch gekennzeichnet, dass** die Fettsäuren gemäß e) gesättigte oder ungesättigte, lineare oder verzweigte einbasische Säuren mit 6 bis 22 C-Atomen sind.

7. Polyglycerinpartialester gemäß den Ansprüchen 1 bis 6, **dadurch gekennzeichnet, dass** der Veresterungsgrad der Polyglycerinmischung zwischen 20 und 75 % der OH-Gruppen liegt.

8. Polyglycerinpartialester gemäß den Ansprüchen 1 bis 7, **dadurch gekennzeichnet, dass** es erhältlich ist durch Veresterung von
a) 1,0 mol OH-Gruppen eines Polyglyceringemisches mit
b) 0,01 bis 0,9 mol COOH-Gruppen einer Polyhydroxystearinsäure und
c) 0,01 bis 0,9 mol COOH-Gruppen von Di- und/oder Tricarbonsäuren und/oder
d) 0 bis 0,9 mol COOH-Gruppen von Dimerfettsäuren, und
e) 0,1 bis 0,9 mol COOH-Gruppen von Fettsäuren mit 6 bis 22 C-Atomen,
mit der Maßgabe, dass die Summe der COOH-Gruppen 20 bis 75 % der OH-Gruppen des Polyglyceringemisches entspricht.

9. Verfahren zur Herstellung der Polyglycerinpartialester gemäß Anspruch 1, **dadurch gekennzeichnet, dass** in
erster Stufe Polyglycerin bis zu einem Veresterungsgrad von 10 bis 70 %, bevorzugt 25 bis 40 %, mit Fettsäure und Di- und/oder Tricarbonsäuren und/oder Dimersäure verestert und in
zweiter Stufe mit Polyhydroxystearinsäure zu einem Gesamtveresterungsgrad 20 bis 75 %, bevorzugt 40 bis 60 %, verestert wird oder dass in
erster Stufe Polyglycerin bis zu einem Veresterungsgrad von 10 bis 70 %, bevorzugt 25 bis 40 %, mit Fettsäure und Polyhydroxystearinsäure verestert und in
zweiter Stufe mit Di- und/oder Tricarbonsäuren und/oder Dimersäure zu einem Gesamtveresterungsgrad 20 bis 75 %, bevorzugt 40 bis 60 %, verestert wird.

10. Verwendung der erfindungsgemäßen Polyglycerinpartialester als W/O-Emulgatoren in kosmetischen oder pharmazeutischen Zubereitungen.

## Claims

1. Polyglycerol partial ester of polyhydroxystearic acid and polyfunctional carboxylic acids, obtainable by esterification of a
a) polyglycerol mixture with
b) polyhydroxystearic acid and
c) di- and/or tricarboxylic acids and optionally/or with
d) dimer fatty acids, and
e) fatty acids having 6 to 22 C atoms.

2. Polyglycerol partial ester according to Claim 1, **characterized in that** the polyglycerol mixture according to a) has an average degree of condensation of 1 to 10.

3. Polyglycerol partial ester according to Claims 1 to 2, **characterized in that** the polyhydroxystearic acid according to b) has an average degree of condensation of 1 to 10.

4. Polyglycerol partial ester according to Claims 1 to 3, **characterized in that** the di- and/or tricarboxylic acids according to c) are aliphatic, linear or branched dicarboxylic acids and contain 2 to 16 C atoms.

5. Polyglycerol partial ester according to Claims 1 to 4, **characterized in that** the dimer fatty acids according to d) have an average functionality of 2 to 3.

6. Polyglycerol partial ester according to Claims 1 to 5, **characterized in that** the fatty acids according to e) are saturated or unsaturated, linear or branched monobasic acids having 6 to 22 C atoms.

7. Polyglycerol partial ester according to Claims 1 to 6, **characterized in that** the degree of esterification of the polyglycerol mixture is between 20 and 75% of the OH groups.

8. Polyglycerol partial ester according to Claims 1 to 7, **characterized in that** is obtainable by esterification of
a) 1.0 mol of OH groups of a polyglycerol mixture with
b) 0.01 to 0.9 mol of COOH groups of a polyhydroxystearic acid and
c) 0.01 to 0.9 mol of COOH groups of di- and/or tricarboxylic acids and/or
d) 0 to 0.9 mol of COOH groups of dimer fatty acids, and
e) 0.1 to 0.9 mol of COOH groups of fatty acids having 6 to 22 C atoms,
with the proviso that the sum of the COOH groups corresponds to 20 to 75% of the OH groups of the polyglycerol mixture.

9. Process for the preparation of the polyglycerol partial esters according to Claim 1, **characterized in that** it comprises, in
a first stage esterification of polyglycerol with fatty acid and di- and/or tricarboxylic acids and/or dimer acid up to a degree of esterification of 10 to 70%, preferably 25 to 40%, and in
a second stage esterification of the product with polyhydroxystearic acid to a total degree of esterification of 20 to 75%, preferably 40 to 60%, or in
a first stage esterification of polyglycerol with fatty acid and polyhydroxystearic acid up to a degree of esterification of 10 to 70%, preferably 25 to 40%, and in
a second stage esterification of the product with di- and/or tricarboxylic acids and/or dimer acid to a total degree of esterification of 20 to 75%, preferably 40 to 60%.

10. Use of the polyglycerol partial esters according to the invention as W/O emulsifiers in cosmetic or pharmaceutical formulations.

## Revendications

1. Ester partiel de polyglycérol avec un poly(acide hydroxystéarique) et des acides carboxyliques polyfonctionnels, pouvant être obtenu par estérification
a) d'un mélange de polyglycérols avec
b) un poly(acide hydroxystéarique) et
c) des acides di- et/ou tricarboxyliques et éventuellement/ou avec
d) des acides gras dimères, et
e) des acides gras ayant de 6 à 22 atomes de carbone.

2. Ester partiel de polyglycérol selon la revendication 1, **caractérisé en ce que** le mélange de polyglycérols selon a) présente un degré moyen de condensation de 1 à 10.

3. Ester partiel de polyglycérol selon les revendications 1 et 2, **caractérisé en ce que** le poly(acide hydroxystéarique) selon b) présente un degré moyen de condensation de 1 à 10.

4. Ester partiel de polyglycérol selon les revendications 1 à 3, **caractérisé en ce que** les acides di- et/ou tricarboxyliques selon c) sont des acides dicarboxyliques aliphatiques, linéaires ou ramifiés et comportent de 2 à 16 atomes de carbone.

5. Ester partiel de polyglycérol selon les revendications 1 à 4, **caractérisé en ce que** les acides gras dimères selon d) présentent une fonctionnalité moyenne de 2 à 3.

6. Ester partiel de polyglycérol selon les revendications 1 à 5, **caractérisé en ce que** les acides gras selon e) sont des acides gras monobasiques saturés ou insaturés, linéaires ou ramifiés ayant de 6 à 22 atomes de carbone.

7. Ester partiel de polyglycérol selon les revendications 1 à 6, **caractérisé en ce que** le degré d'estérification du mélange de polyglycérols se situe entre 20 et 75 % des groupes OH.

8. Ester partiel de polyglycérol selon les revendications 1 à 7, **caractérisé en ce qu'**il peut être obtenu par estérification de
a) 1,0 mole de groupes OH d'un mélange de polyglycérols avec
b) 0,01 à 0,9 mole de groupes COOH d'un poly(acide hydroxystéarique) et
c) 0,01 à 0,9 mole de groupes COOH d'acides di- et/ou tricarboxyliques et/ou
d) 0 à 0,9 mole de groupes COOH d'acides gras dimères, et
e) 0,1 à 0,9 mole de groupes COOH d'acides gras ayant de 6 à 22 atomes de carbone,
étant entendu que la somme des groupes COOH correspond à 20 à 75 % des groupes OH du mélange de polyglycérols.

9. Procédé pour la préparation des esters partiels de polyglycérols selon la revendication 1, **caractérisé en ce que** dans
une première étape on estérifie du polyglycérol jusqu'à un degré d'estérification de 10 à 70 %, de préférence de 25 à 40 %, avec un acide gras et des acides di-et/ou tricarboxyliques et/ou un acide dimère et dans
une deuxième étape on l'estérifie avec un poly(acide hydroxystéarique) jusqu'à un degré d'estérification total de 20 à 75 %, de préférence de 40 à 60 % ou **en ce que** dans
une première étape on estérifie du polyglycérol jusqu'à un degré d'estérification de 10 à 70 %, de préférence de 25 à 40 %, avec un acide gras et un poly(acide hydroxystéarique) et dans
une deuxième étape on l'estérifie avec des acides di-et/ou tricarboxyliques et/ou un acide dimère jusqu'à un degré d'estérification total de 20 à 75 %, de préférence de 40 à 60 %.

10. Utilisation des esters partiels de polyglycérol selon l'invention en tant qu'émulsifiants E/H dans des préparations cosmétiques ou pharmaceutiques.
